# Europäisches Patentamt
## European Patent Office
## Office européen des brevets

(19)

(11) Veröffentlichungsnummer: **0 163 121**
**B1**

(12) **EUROPÄISCHE PATENTSCHRIFT**

(45) Veröffentlichungstag der Patentschrift:
**10.08.88**

(51) Int. Cl.⁴: **A 61 F 2/36**, A 61 F 2/30

(21) Anmeldenummer: **85104966.8**

(22) Anmeldetag: **24.04.85**

(54) Anordnung zum Herstellen einer anatomisch angemessenen Endoprothese.

(30) Priorität: **11.05.84 DE 3417609**

(43) Veröffentlichungstag der Anmeldung:
**04.12.85 Patentblatt 85/49**

(45) Bekanntmachung des Hinweises auf die Patenterteilung:
**10.08.88 Patentblatt 88/32**

(84) Benannte Vertragsstaaten:
**AT BE CH DE FR GB IT LI NL SE**

(56) Entgegenhaltungen:
**EP-A-0 000 549**
**EP-A-0 079 441**
**WO-A-83/03049**
**DE-A-2 012 800**
**DE-A-3 205 577**
**DE-B-1 122 364**
**DE-B-2 729 853**
**SU-A-171 970**
**US-A-1 840 703**
**US-A-2 557 669**
**US-A-4 195 368**
**US-A-4 506 393**

**BIOMEDIZINISCHE TECHNIK, Band 26, Heft 1-2, Januar-Februar 1981, Seiten 19-27; C.H. SIEMSEN: "Systematik der Entwicklung einer Endoprothese von der Planung bis zur Implantation, dargestellt am Beispiel der Sprunggelenk-Endoprothese,**

(73) Patentinhaber: **Waldemar Link GmbH & Co,**
**Barkhausenweg 10, D-2000 Hamburg 63 (DE)**

(72) Erfinder: **Link, Helmut D., Wildstieg 14, D-2000 Hamburg 65 (DE)**

(74) Vertreter: **Glawe, Delfs, Moll & Partner Patentanwälte, Postfach 26 01 62 Liebherrstrasse 20, D-8000 München 26 (DE)**

(56) Entgegenhaltungen: (Fortsetzung)
**Modell BME"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Anordnung zum Herstellen einer anatomisch angemessenen Endoprothese mit einem in dem sich erweiternden Ende eines Röhrenknochens zu verankernden Schaft, insbesondere einer im Oberschenkelknochen zu verankernden Gelenk-Schaftprothese, unter Verwendung von maßstabsgerechten Röntgenaufnahmen, einem Satz von einer beschränkten Anzahl Prothesenschablonen und einem Satz entsprechender Prothesen-Herstellungsmodelle.

Es ist bekannt, daß man eine Endoprothese für einen bestimmten Patienten dadurch in angemessener Form auswählen kann, daß man Schablonen, die die Gestalt der Prothese in der AP- und der LM-Ebene wiedergeben mit maßstabsgerechten Röntgenaufnahmen in der AP- bzw. LM-Ebene durch Übereinanderlegen vergleicht. Naturgemäß sind jedoch die individuellen Anpassungsmöglichkeiten begrenzt, wenn Modelle und Schablonen nur von einer beschränkten Anzahl von vollständigen Prothesen vorhanden sind. Dem zunehmenden Bestreben nach individueller Anpassung, insbesondere auch unter dem Blickpunkt der zementfreien Verankerung, vermag man auf diese bekannte Weise nicht mehr gerecht zu werden.

Es ist bekannt (EP-A-0 093 869), individuell angepaßte Endoprothesen dadurch herzustellen, daß von dem mit der Endoprothese auszustattenden Knochen durch Computer-Tomographie oder eine vergleichbare Technik ein vollständiges Raumbild hergestellt und davon rechnergestützt die angemessene Endoprothesengestalt abgeleitet wird. Dieses Verfahren ist jedoch sehr aufwendig und nur in Verbindung mit ungewöhnlich gut ausgestatteten Kliniken durchführbar.

Der Erfindung liegt daher die Aufgabe zugrunde, eine Anordnung der eingangs genannten Art zu schaffen, die die Herstellung anatomisch angemessener Prothesen auf einfachere Weise gestattet.

Die erfindungsgemäße Lösung besteht darin, daß ein Satz von Teilschablonen für den in dem die stärkste Erweiterung aufweisenden Endabschnitt des Knochens zu verankernden Teil des Prothesenschafts sowie eine Einrichtung zur Bestimmung charakteristischer Maße der an diesem Abschnitt angrenzenden Abschnitte des Knochens bzw. der entsprechenden Prothesenmaße, nämlich mindestens einer Dicke des auf der der Erweiterung entgegengesetzten Seite angrenzenden Abschnitts, vorgesehen ist und daß ein den Teilschablonen entsprechender Satz von Herstellungsmodellen vorgesehen ist, die mit Herstellungsmodellen für die an den erwähnten Teil des Prothesenschafts angrenzenden Prothesenabschnitte verbindbar sind.

Die Erfindung geht von der Erfahrung aus, daß bestimmte Abschnitte des den Prothesenschaft aufnehmenden Knochenteils jeweils für sie charakteristische Formvariationen aufweisen, wobei diese Variationen in dem einen Abschnitt weitgehend unabhängig von denen im anderen Abschnitt sind. Dabei treten die stärksten Formunterschiede in dem endnahen, die stärkste Erweiterung bildenden Abschnitt des Knochens auf, nämlich bei einem Oberschenkelknochen etwa im Bereich oberhalb der unteren Grenzlinie des kleineren Trochanter. Ferner geht die Erfindung von der Erkenntnis aus, daß es durchaus nicht notwendig ist, eine individuelle Anpaßbarkeit in unendlicher Feinheit anzustreben, sondern daß eine Stufung angesichts der ohnehin unvermeidbaren oder tolerierbaren Ungenauigkeiten vertretbar ist. Dabei besteht die Erfindung wesentlich in der Erkenntnis, daß die Zahl der Modelle und der ihnen zugeordneten Schablonen verhältnismäßig klein gehalten werden kann, sofern man die jeweils anatomisch angemessensten Maße und Formen in den einzelnen, charakteristischen Knochenbereichen gesondert festlegt.

Die mit der Schablone verbundenen Mittel zur Bestimmung der Maße der anschließenden Prothesenteile werden zweckmäßigerweise ebenfalls von je einem Satz von Teilschablonen gebildet, die als Neben-Teilschablonen die zuvor genannten Haupt-Teilschablonen ergänzen. Auf diese Weise ist es möglich, die Gestalt einer Prothese durch Zusammensetzung unterschiedlich gestalteter Schablonenabschnitte zu bestimmen. Die Schablonen dienen dabei nicht nur zur Festlegung der Durchmesser und Längenmaße der betrachteten Prothesenabschnitte, sondern müssen auch einen Hinweis auf ihre Richtung im Verhältnis zur Richtung der Hauptschablone geben. Insofern müßten Sie richtungsbestimmt mit der Haupt-Teilschablone verbindbar sein, d.h. ihre Richtung muß sich beziehen lassen auf eine vorbestimmte Bezugsrichtung der Haupt-Teilschablone. Diese Richtungsbestimmtheit wird am einfachsten dadurch verwirklicht, daß die Haupt-Teilschablonen und die Neben-Teilschablonen durch zusammenwirkende Führungsmittel richtungsfest miteinander verbunden bzw. verbindbar sind. Unbedingt erforderlich ist dies jedoch nicht. Beispielsweise können sie an der Grenzstelle verschwenkbar miteinander verbunden sein, wobei der jeweils ausgewählte Winkel durch den Winkel der Längsrichtung der Neben-Teilschablone gegenüber einer durch die Haupt-Teilschablone festgelegten Bezugsrichtung angegeben werden kann. Indessen ist die richtungsfeste Zusammenwirkung der Schablonen über Führungsmittel schon deshalb vorzuziehen, weil dadurch Fehler bei der Bestimmung der Prothesenform mit größerer Sicherheit ausgeschlossen werden können.

Die zusammenwirkenden Führungsmittel der Teilschablonen sind im einfachsten Falle gerade Kanten, längs denen die Schablonen aneinandergelegt werden können. Besonders zweckmäßig und einfach ist eine Ausführung, bei der die Teilschablonensätze auf parallel zueinander verschiebbaren Trägerstreifen angeordnet sind. Die ausgedehnten Längskanten dieser Trägerstreifen bilden aneinandergelegt sichere Führungskanten. Ferner besteht die Möglichkeit, die Teilschablonen bzw. Teilschablonensätze in einem mit entsprechenden mechanischen Führungsmitteln ausgestatteten Gerät, welches zweckmäßigerweise auch eine Halterung für das zugehörige Röntgenbild bildet, anzuordnen.

Im Falle einer Hüftgelenk-Oberschenkelschaftprothese hat die dem proximalen Schaftabschnitt zugeordnete Teilschablone zweckmäßigerweise eine mittlere Länge von etwa 8 cm, gemessen vom proximalen Schaftende bis zu ihrem die Grenze zur nächsten Schablone bildenden distalen Ende. Ferner ist es zweckmäßig, wenn das distale Ende dieser Teilschablone, also die Grenze zu dem nächsten Prothesenschaftabschnitt, in dem Bereich des Übergangs von der proximalen Krümmung mit vorne gelegenem Krümmungsmittelpunkt zu der sich distal anschließenden Krümmung mit hinten gelegenem Krümmungsmittelpunkt liegt. Es hat sich zum einen gezeigt, daß dabei der proximale Teil in welchem die stärksten individuellen Maßunterschiede auftreten, durch diese Begrenzung besonders gut umrissen wird. Zum anderen läßt sich auf diese Weise eine optimale Anpassung bei unterschiedlichen Krümmungsverhältnissen des Knochens finden. Für eine angemessene Definition eines Hüftgelenkprothesenschafts genügen dann im allgemeinen zwei dem Prothesenschaft zugeordnete Schablonensätze.

Sobald die Schablonen zu den Röntgenbildern passend ausgesucht sind, läßt sich daraus die dem Knochen zugeordnet, innerhalb der vorgesehenen Toleranzen individuell exakt passende Prothese entwickeln, indem die den Schablonen zugehörigen Herstellungsmodelle zusammengesetzt und zum Guß einer entsprechenden Prothese verwendet werden.

Um dem Arzt eine Vorstellung davon zu ermöglichen, wie die von ihm zusammengesetzte Prothese aussehen wird, können Sätze von den Schablonen bzw. Herstellungsmodellen entsprechenden Probeprothesenabschnitten vorgesehen sein, die mit Einrichtungen zum formschlüssigen Zusammensetzen versehen sind. Der Arzt kann dann daraus eine Probeprothese entsprechend den von ihm für richtig gehaltenen Schablonen zusammensetzen und sich anhand der räumlichen Anschauung von der Richtigkeit seiner Wahl überzeugen.

Die Erfindung wird im folgenden näher unter Bezugnahme auf die Zeichnung erläutert, die vorteilhafte Ausführungsbeispiele veranschaulicht. Darin zeigen:

Fig. 1    eine erste Ausführungsform eines Schablonenblatts mit unterschiedlichen Teilschablonen in der LM-Ebene,

Fig. 2    ein dem Schablonenblatt gemäß Fig. 1 zugehöriges Schablonenblatt in der AP-Ebene,

Fig. 3    in der linken vertikalen Reihe einen Satz von Schablonen der proximalen Femurschaftabschnitte in der LM-Ebene und in der rechten vertikalen Reihe in der AP-Ebene,

Fig. 4    zwei der Fig. 3 entsprechende und zugehörige Sätze der distalen Femurschaftabschnitte,

Fig. 5    ein aus drei Streifen bestehendes Schablonenblatt für eine Hüftgelenk-Femurschaftprothese in der Darstellung in der LM-Ebene,

Fig. 6    ein der Fig. 5 ähnliches Schablonenblatt mit an Führungen verschiebbaren Teilschablonen,

Fig. 7    eine Probeprothese

Fig. 8    die Verbindungsanordnungen zwischen zwei Teilen der Probeprothese und

Fig. 9    einen Teile-Kasten für Probeprothesen.

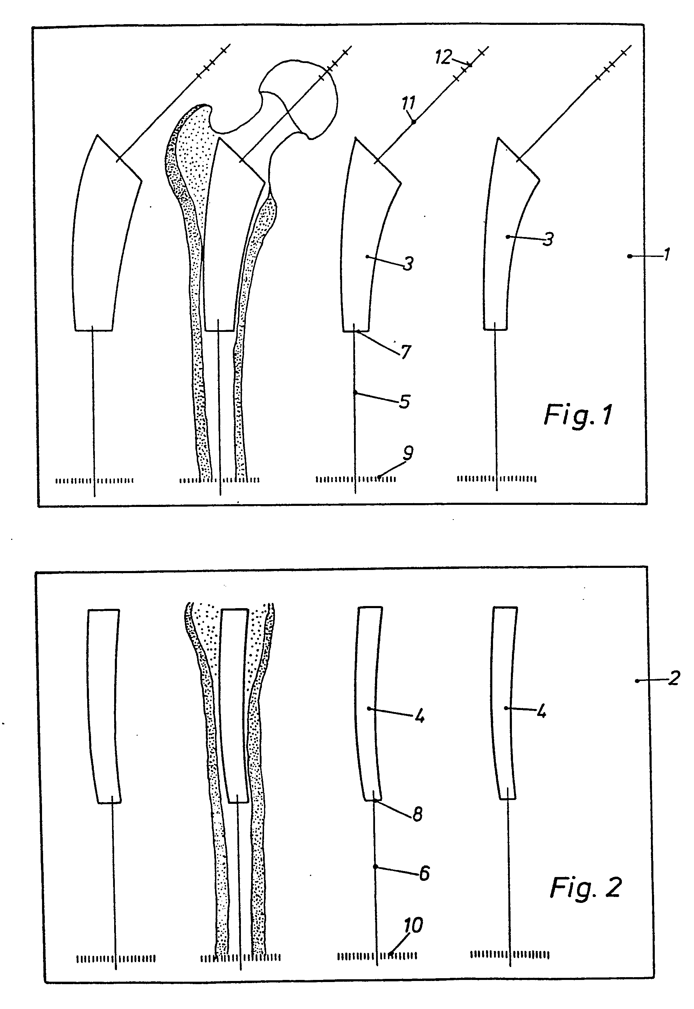

Auf den Schablonenblättern 1 und 2 gemäß Fig 1 und 2 erkennt man einen Satz von Teilschablonen des proximalen Abschnitts des Schafts einer Hüftgelenk-Schaftprothese in der LM- bzw. AP-Ebene. Die Dicken- und Krümmungsgrade dieser Prothesenabschnitte weisen eine sämtliche praktischen Verhältnisse mit hinreichender Feinheit wiedergebende Abstufung auf. Selbstverständlich kann eine größere Zahl von Variationen vorgesehen sein als die dargestellten vier. Die Blätter sind durchsichtig, so daß ihre Darstellungen mit maßstabsgerechten Röntgenbildern in Übereinstimmung gebracht werden können, wie dies in Fig. 1 und 2 jeweils beiden zweiten Teilschablonen von links angedeutet ist. Der Arzt kann so die für den jeweils vorliegenden Fall passende Teilschablone auswählen. Jede Teilschablone 3 bzw. 4 ist mit einem nach distal weisenden Richtungszeiger 5 bzw. 6 verbunden, der in einigen Zentimeter Abstand von der unteren Endkante 7 bzw. 8 eine seitliche Strichteilung 9 bzw. 10 aufweist. Diese Strichteilung befindet sich an einer für die Dicke des distalen Prothesenschaftabschnitts charakteristischen Stelle, beispielsweise im Bereich zwischen seiner Mitte und seinem distalen Ende. Die Ablesung an dieser Strichteilung gestattet sowohl die Festlegung der Dicke als auch der Richtung des distalen Prothesenschaftabschnitts. Charakterisiert man beispielsweise bei der zweiten Schablone von links in Fig. 1 den distalen Schaftabschnitt mit "zwei Strich links, vier Strich rechts", so ist damit sowohl die Dicke des Schafts in dieser Darstellungsebene mit sechs Strich als auch die Richtung mit ein Strich rechts festgelegt. - Ferner ist mit der Schablone 3 ein Richtungszeiger 11 verbunden mit einer Strichteile 12 zur Festlegung der Kopf-Hals-Länge.

Der Prothesenhersteller verfügt über einen Satz vorgefertigter Gußmodelle für den proximalen Schaftabschnitt, der durch die Teilschablonen 3 bzw. 4 repräsentiert ist, sowie über Sätze von Gußmodellen für die angrenzenden Prothesenabschnitte, die nach den Dicken- und Richtungsangeben ausgewählt und mit den Haupt-Teilmodellen zur Bildung des vollständigen Gußmodells verbunden werden.

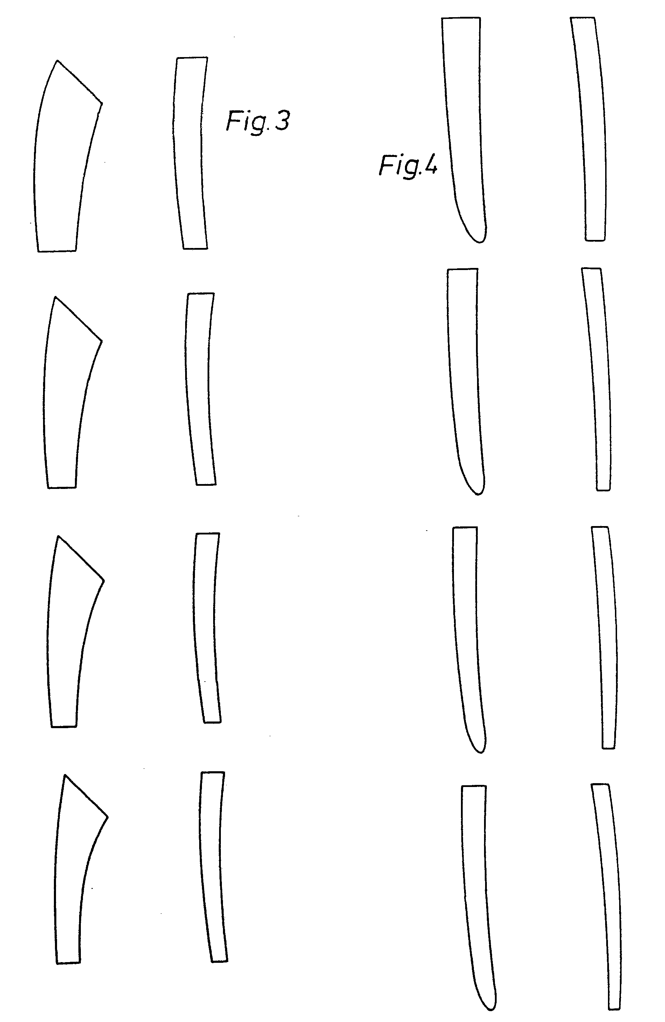

Statt die Maße und Richtungen der an den Haupt-Teilabschnitt der Prothese angrenzenden Abschnitte lediglich durch Zahlen gemäß Fig. 1 und 2 zu identifizieren, können dafür auch Teilschablonen vorgesehen sein. Fig. 3 zeigt einen Satz von Haupt-Teilschablonen für den proximalen Abschnitt einer Oberschenkelschaftprothese in der LM-Darstellung (links) und der AP-Darstellung (rechts). Diese Darstellungen entsprechen denen in Fig. 1 und 2. Entsprechende Darstellungen (LM-Ebene links, AP-Ebene rechts) zeigt Fig. 4 von einem Satz von Neben-Teilschablonen für den distalen Schaftabschnitt. Man erkennt, daß man die gesamte Schaftform durch Zusammenfügen jeweils einer Haupt- und einer Neben-Teilschablone in der jeweiligen Darstellungsebene nachbilden kann. Die Ausrichtung der Schablonen gegeneinander erfolgt durch spaltfreies Aneinanderlegen an ihren geraden Grenzkanten. - Der Prothesenhersteller verfügt wieder über einen entsprechenden Satz von Gußmodellen, wobei etwaige Durchmesserunterschiede im Grenzbereich ausgeglichen werden können. In den in Fig. 3 und 4 jeweils rechts gezeigten Darstellungen in der AP-Ebene erkennt man, daß der proximale Abschnitt (Fig. 3) eine Krümmung mit vorne gelegenem Krümmungsmittelpunkt und der untere Abschnitt eine Krümmung mit hinten gelegenem Krümmungsmittelpunkt aufweist und daß die Grenze zwischen den beiden Teilschablonen bzw. Modellen im Bereich des Kürmmungs-Wendepunkts liegt. Dieser liegt bei allen Menschen im Bereich von etwa 4 cm bis 11 cm unterhalb der Ebene Oberkante Trochanter major/Oberkante Trochanter minor. Etwaige Krümmungsverschiebungen in diesem Bereich sind praktisch belanglos, weil der Verlauf der Markhöhle hier annähernd gerade ist.

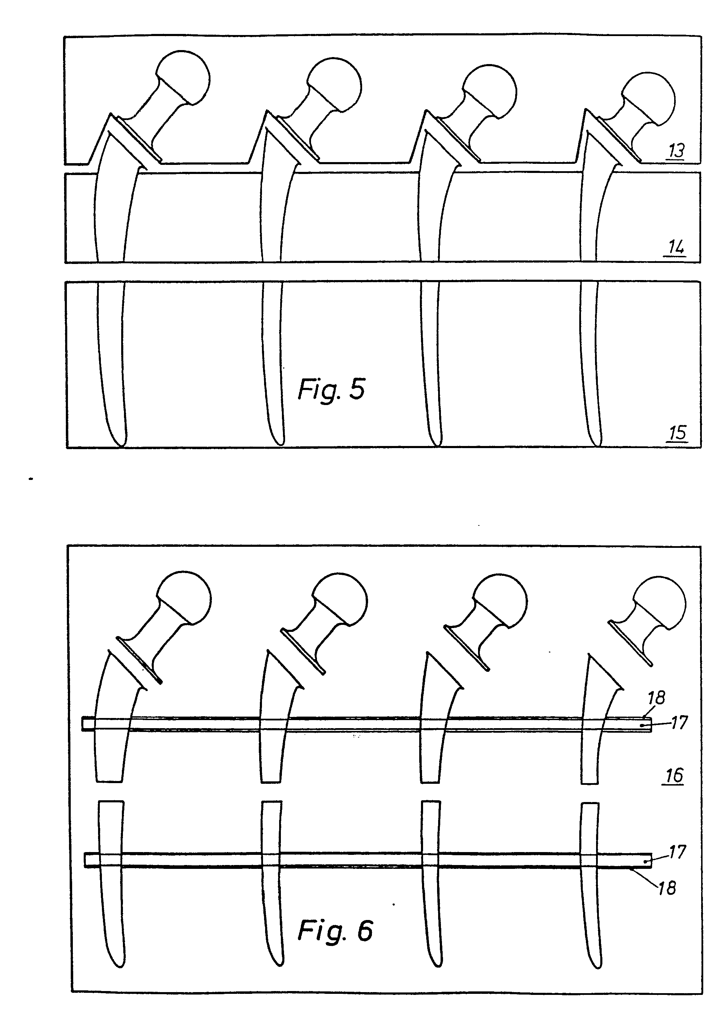

Da die Benutzung einzelner Teilschablonen, wie sie in Fig. 3 und 4 dargestellt sind, umständlich ist, sieht Fig. 5 die Zusammenstellung von dem gleichen Abschnitt zugeordneten Teilschablone jeweils in einem Schablonenstreifen vor. Der Schablonenstreifen 13 zeigt die Kopf-Hals-Partie mit unterschiedlichen Längen. Der Streifen 14 enthält die Teilschablonen für den oberen Schaftbereich und der Abschnitt 15 zeigt die Teilschablonen für den unteren Schaftabschnitt. Da diese Streifen ausgedehnte gerade Grenzkanten haben, ergibt sich im praktischen Gebrauch eine gute gegenseitige Führung, so daß Winkelfehler beim Zusammenfügen der einzelnen Schablonen so gut wie ausgeschlossen sind. - Selbstverständlich gehört zu der Schablone gemäß Fig. 5, die die Darstellungen in der LM-Ebene enthält, eine entsprechende Schablone für die Darstellungen in der AP-Ebene, die der Einfachheit halber nicht gezeigt wurde.

Eine der Fig. 5 ähnliche Anordnung zeigt Fig. 6. Auf dem Schablonenblatt 16 sind die Kopf-Hals-Partien fest dargestellt, während die Teilschablonen für den Schaft an Plastikschienen 17 befestigt sind, die ihrerseits in entsprechenden Führungen 18 des Schablonenblatts 16 verschiebbar sind.

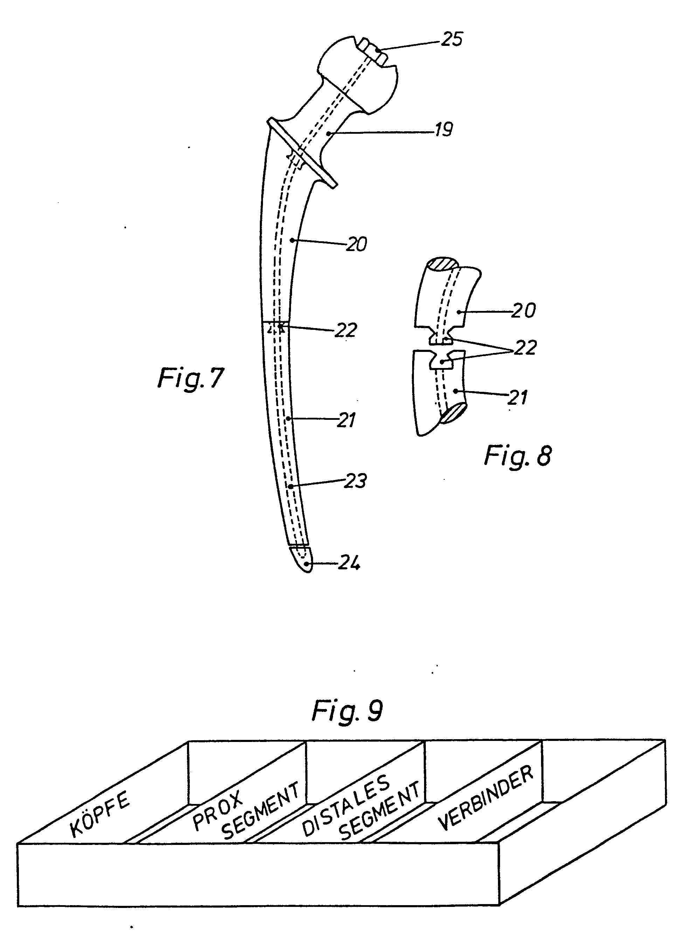

Die Probeprothese gemäß Fig. 7 setzt sich aus Abschnitt 19, 20, 21 zusammen, die, wie Fig. 8 in etwa naturgroßer Darstellung veranschaulicht, mit Schwalbenschwanz-Verbindugsprofilen 22 ausgerüstet sind, mit deren Hilfe sie richtungsfest zusammengefügt werden können. Um ein Auseinandergleiten zu verhindern, sind die Abschnitte in Längsrichtung durchbohrt und werden sie von einem flexiblen Zugelement 23, das am einen Ende mit der Prothesenschaftspitze 24 fest verbunden ist und am anderen Ende durch eine Mutter 25 gespannt werden kann, zusammengehalten.

Zum Zusammenstellen solcher Probeprothesen sind Sätze von jeweils den Teilschablonen entsprechenden Prothesenabschnitten vorgesehen und in einem beispielsweise gemäß Fig. 9 gestalteten Behältnis zusammengestellt.

## Patentansprüche

1. Anordnung zum Herstellen einer anatomisch angemessenen Endoprothese mit einem in dem sich erweiternden Ende eines Röhrenknochens zu verankernden Schaft, insbesondere einer im Oberschenkelknochen zu verankernden Hüftgelenk-Schaftprothese, unter Verwendung von maßstabsgerechten Röntgenaufnahmen, einem Satz von einer beschränkten Anzahl von Prothesenschablonen und einem Satz entsprechender Prothesenherstellungsmodelle, dadurch gekennzeichnet, daß ein Satz von Teilschablonen (3, 4) für den in dem die stärkste Erweiterung aufweisenden Endabschnitt des Knochens zu verankernden Teil des Prothesenschafts sowie eine Einrichtung zur Bestimmung charakteristischer Maße der an diesen Abschnitt angrenzenden Abschnitte des Knochens bzw. der entsprechenden Prothesenmaße, nämlich mindestens einer Dicke des auf der der Erweiterung entgegengesetzten Seite angrenzenden Abschnitts, vorgesehen ist und daß ein den Teilschablonen (3, 4) entsprechender Satz von Herstellungsmodellen vorgesehen ist, die mit Herstellungsmodellen für die an den erwähnten Teil des Prothesenschafts angrenzenden Prothesenabschnitte verbindbar sind.

2. Anordnung nach Anspruch 1, dadurch gekennzeichnet, daß die Maßbestimmungsmittel ebenfalls von je einem Satz von Teilschablonen gebildet sind.

3. Anordnung nach Anspruch 2, dadurch gekennzeichnet, daß die Teilschablonen durch zusammenwirkende Führungsmittel richtungsfest miteinander verbunden bzw. verbindbar sind.

4. Anordnung nach einem der Ansprüche 1 bis 3, dadurch gekennzeichnet, daß die dem proximalen Abschnitt einer Oberschenkelschaftprothese zugeordnete Teilschablone eine mittlere Länge von etwa 8 cm vom proximalen Schaftende bis zu ihrem distalen Ende aufweist.

5. Anordnung nach einem der Ansprüche 1 bis 4, dadurch gekennzeichnet, daß das distale Ende der dem oberen Abschnitt einer Oberschenkelschaftprothese zugeordneten Teilschablone in dem Bereich des Übergangs von der proximalen Krümmung mit vorne gelegenem Krümmungsmittelpunkt zu der sich distal anschließenden Krümmung mit hinten gelegenem Krümmungsmittelpunkt liegt.

6. Anordnung nach einem der Ansprüche 2 bis 5, dadurch gekennzeichnet, daß die Teilschablonensätze auf parallel zueinander verschiebbaren Trägerstreifen angeordnet sind.

7. Anordnung nach einem der Ansprüche 2 bis 6, dadurch gekennzeichnet, daß zwei oder mehrere dem Prothesenschaft zugeordnete Schablonen vorgesehen sind.

8. Anordnung nach einem der Ansprüche 1 bis 7, dadurch gekennzeichnet, daß zur Bildung einer Probeprothese den Teilschablonen formentsprechende formschlüssig zusammensetzbare Sätze von Prothesenabschnitten vorgesehen sind.

## Claims

1. An arrangement for producing an anatomically made to measure endoprosthesis, with a shaft to be anchored in the widened end of a hollow bone, in particular a hip-joint shaft prosthesis to be anchored in the femur, utilising X-ray photographs which are true to scale, a set of a limited number of prosthesis templates and a set of corresponding prosthesis production patterns, characterised by the provision of a set of partial templates (3, 4) for that part of the prosthesis shaft to be anchored in the end segment of the bone having the greatest widening, and means for determining characteristic dimensions of segments of the bone adjoining this segment or the corresponding prosthesis dimensions, namely at least the thickness of an adjacent segment on the side opposite the widening, and by the provision of a set of production patterns corresponding to the partial templates (3, 4), which can be joined to production patterns for the prosthesis segments adjoining said part of the prosthesis shaft.

2. An arrangement according to Claim 1, characterised in that the means for determining dimensions also comprise a respective set of partial templates.

3. An arrangement according to Claim 2, characterised in that the partial templates are joined or can be joined together in a directionally stable manner by cooperating guide means.

4. An arrangement according to any one of Claims 1 to 3, characterised in that the partial template associated with the proximal segment of a femur shaft prosthesis has an average length of about 8 cm from the proximal shaft end up to its distal end.

5. An arrangement according to any one of Claims 1 to 4, characterised in that the distal end of the partial template associated with the upper segment of a femur shaft prosthesis lies in the vicinity of the transition from the proximal curvature with a forwardly located centre of curvature into the distally adjoining curvature with a rearwardly located centre of curvature.

6. An arrangement according to any one of Claims 2 to 5, characterised in that the sets of partial templates are mounted on support strips which are movable parallel to one another.

7. An arrangement according to any one of Claims 2 to 6, characterised in that two or more templates associated with the prosthesis shaft are provided.

8. An arrangement according to any one of Claims 1 to 7, characterised in that sets of prosthesis segments, which can be assembled in form-locking manner to form a sample prosthesis conforming to the shape of the partial templates, are provided.

## Revendications

1. Dispositif pour la fabrication d'une endoprothèse sur mesures anatomiques, comportant une tige destinée à être ancrée dans l'extrémié évasée d'un os long, en particulier pour la fabrication d'une prothèse de hanche à tige destinée à être ancrée dans le fémur, avec utilisation de clichés radiographiques à l'échelle, d'un jeu d'un nombre limité de gabarits de prothèse et d'un jeu de modèles de fabrication de prothèse correspondants, caractérisé en ce qu'il est prévu un jeu de gabarits partiels (3, 4) pour la partie de la tige de prothèse qui doit être ancrée dans le segment terminal de l'os présentant l'evasement le plus prononcé, ainsi qu'un dispositif pour la détermination de dimensions caractéristiques des segments de l'os contigus à ce segment ou des dimensions correspondantes de la prothèse, à savoir au moins une épaisseur du segment contigu du côté opposé à l'évasement, et en ce qu'il est prevu un jeu de modèles de fabrication qui correspondent aux gabarits partiels (3, 4) et qui péuvent être joints à des modèles de fabrication pour les segments de la prothèse contigus à ladite partie de la tige de prothèse.

2. Dispositif selon la revendication 1, caractérisé en ce que les moyens de détermination des mesures sont également consitués par un jeu de gabarits partiels.

3. Dispositif selon la revendivation 2, caractérisé en ce que les gabarits partiels sont assembles ou assemblablés entre eux avec une orientation fixe par des moyens de guidage qui coopèrent.

4. Dispositif selon l'une quelconque des revendications 1 à 3, caractérisé en ce que le gabarit partiel correspondant au segment proximal d'une prothèse fémorale présente une longueur moyenne d'environ 8 cm depuis l'extrémité proximale de la tige jusqu'à son extrémité distale.

5. Dispositif selon l'une quelconque des revendications 1 à 4, caractérisé en ce que l'extrémité distale du gabarit partiel correspondant au segment supérieur d'une prothèse fémorale se trouve dans la région de la transition entre la courbure proximale à centre de courbure situé en avant et la courbure qui lui fait suite dans le sense distal et dont le centre de courbure est situé en arrière.

6. Dispositif selon l'une quelconque des revendications 2 à 5, caractérisé en ce que les jeux de gabarits partiels son disposés sur des bandes de support qui peuvent être déplacées parallèlement les unes aux autres.

7. Dispositif selon l'une quelconque des revendications 2 à 6, charactérisé en ce qu'il est prévu deux ou plusieurs gabarits correspondant à la tige de prothèse.

8. Dispositif selon l'une quelconque des revendications 1 à 7, caractérisé en ce qu'il est prévu, pour la formation d'une prothèse d'essai, des jeux de segments de prothèse qui peuvent être assemblés par emboîtement de manière à présenter une forme correspondant à celle des gabarits partiels.

Fig. 1

Fig. 2

Fig. 3

Fig. 4

Fig. 5

Fig. 6

*Fig.7*

*Fig. 8*

*Fig. 9*